Europäisches Patentamt

European Patent Office

Office européen des brevets

(19)

(11) Veröffentlichungsnummer: **0 168 513**
**A1**

## (12) EUROPÄISCHE PATENTANMELDUNG

(21) Anmeldenummer: **84108449.4**

(22) Anmeldetag: **18.07.84**

(51) Int. Cl.⁴: **A 61 N 1/16**

(43) Veröffentlichungstag der Anmeldung: **22.01.86**
**Patentblatt 86/4**

(71) Anmelder: **Rudhardt, Elfriede, Hohe Gasse 2, D-8960 Kempten (DE)**

(72) Erfinder: **Rudhardt, Elfriede, Hohe Gasse 2, D-8960 Kempten (DE)**

(84) Benannte Vertragsstaaten: **AT BE CH DE FR GB IT LI LU NL SE**

(74) Vertreter: **Hübner, Hans-Jürgen, Dipl.-Ing., Mozartstrasse 21, D-8960 Kempten (DE)**

(54) **Einrichtung zum Sammeln von kosmobiologischer Energie.**

(57) Ein Hohlkörper (10) in Form einer regelmäßigen Pyramide mit quadratischem Grundriß trägt einen längs seiner einen Bodenkante (14) anschließenden äußeren Ansatz (18), auf dem ein Magnetkompaß (20) befestigt ist und wobei eine Markierung (24; 25) auf dem Ansatz (18) vorgesehen ist, die mit der Kompaßachse in einer Ebene liegt, welche zu einem Paar Bodenkanten (14) parallel und zum anderen Paar Bodenkanten rechtwinklig liegt.

Der Hohlkörper (10) lässt sich schnell und genau in eine Stellung bringen, in welcher zwei parallele Bodenkanten (14; 16) in Nord-Süd-Richtung der Erde ausgerichtet sind. In dieser Stellung ist die Hohlpyramide in der Lage kosmobiologische Energie zu sammeln und an in ihr befindliche Gegenstände abzugeben.

— 1 —

Die Erfindung betrifft eine Einrichtung zum Sammeln von Energie, bestehend aus einem Hohlkörper in Form einer unten offenen, regelmäßigen Pyramide mit quadratischem Grundriß.

Es ist bekannt, daß Hohlkörper in Form regelmäßiger Pyramiden dann, wenn sie genau mit zwei ihrer Bodenkanten in Nord-Süd-Richtung der Erde ausgerichtet sind, in der Lage sind, kosmobiologische Energie zu sammeln und diese auf Gegenstände innerhalb des Hohlkörpers zu übertragen. Solche Gegenstände laden sich mit der gespeicherten Energie auf und sind in der Lage, sie an Lebewesen abzugeben (indirekte Methode). Als solche Gegenstände kommen nur beispielsweise infrage Becher mit Trinkwasser, Säften, Tees, weiterhin Lebensmittel, aber auch am Körper zu tragender Schmuck, Ringe, Armbänder, Ketten und dergl. Auch für die prophylaktische und therapeutische Behandlung von Pflanzen wird die Einrichtung mit Erfolg eingesetzt. Es ist erwiesen, daß Leitungswasser, das in einem Gefäß in diesem speziellen Hohlkörper für eine gewisse Zeit aufbewahrt worden war, mit Erfolg zu Gießzwecken für Schnittblumen, Gartenblumen und -pflanzen sowie Samen und Setzpflanzen verwendet wurde, wobei diese Pflanzen sehr kräftig gediehen und solche, die von Schädlingen befallen waren, wieder gesundeten. Für den Menschen wirken die unter dem pyramidenförmigen Hohlkörper aufbewahrten
Gegenstände je nach dem gewollten Zweck aktivierend oder
auch beruhigend. Schlaflosigkeit bei Personen konnte nachweisbar in vielen Fällen beseitigt werden, wenn diese
Personen vor dem Schlafengehen ein Glas Wasser tranken,
das etwa 12 Stunden unter dem pyramidenförmigen Hohlkörper gestanden hat.

Der pyramidenförmige Hohlkörper eignet sich auch zur direkten Energieübertragung auf Menschen, Tiere und Pflanzen. Der beste Wirkungsgrad wird erreicht, wenn sich diese
Lebewesen eine Zeitlang im Hohlkörper aufhalten. Erfahrungen mit Samen und Setzpflanzen haben gezeigt, daß
diese, wenn sie einige Tage vor dem Aussäen bzw. vor dem
Setzen unter dem Hohlkörper aufbewahrt wurden, später im
Erdreich kräftiger gediehen als vergleichbare Samen und
Pflanzen, die in herkömmlicher Weise behandelt wurden.

Für die Funktion, kosmobiologische Energie zu sammen, ist
es allerdings unerläßlich, daß der pyramidenförmige Hohlkörper zur Erdachse genau ausgerichtet aufgestellt wird.
Dazu kann man sich eines üblichen Kompasses bedienen. Kompasse mit einem Rechteckgehäuse, deren Nord-Süd-Markierung
parallel oder rechtwinklig zu einer der Gehäuseränder verläuft, könnten an der Bodenkante des pyramidenförmigen
Hohlkörpers angelegt werden, um die aus Hohlkörper und KOmpaß bestehende Einheit in die Nord-Süd-Richtung der Erde oder rechtwinklig dazu zu drehen. Die Handhabung ist aber unpraktisch, weil zwei Fehlermöglichkeiten nebeneinander auftreten können und bemerkt werden müssen, einmal die exakte Nord-Süd-Ausrichtung des Kompasses und zum anderen die spaltfreie Anlage des Kompaßgehäuses an der Hohlkörperbodenkante. Bei größeren Hohlkörpern mit entsprechendem Gewicht benötigt man beide Hände zum Einrichten des Hohlkörpers. Dieser muß zuerst in eine grob geschätzte Position gebracht werden, die dann durch Anlegen des Kompasses überprüft wird, wonach wiederum beide Hände nötig sind, um den Hohlkörper wiederum durch Schätzung besser auszurichten usw., so daß man sich stufenweise an die richtige Stellung herantasten muß, eine Handhabung, die äußerst umständlich, zeitaufwendig ist und häufig noch ein ungenaues Ergebnis bringt. Normale, einfache Magnetkompasse sind schon aufgrund ihrer funktionelle Bauweise von kreisförmiger Gestalt. Mit diesen Kompassen ist ein exaktes Ausrichten des pyramidenförmigen Hohlkörpers überhaupt nicht möglich.

Aufgabe der Erfindung ist es, eine Einrichtung der eingangs genannten Art zu schaffen, mit der der pyramidenförmige Hohlkörper schneller und genauer in die richtige Position gebracht werden kann, die für seine Funktion unabdingbar ist.

Diese Aufgabe wird erfindungsgemäß dadurch gelöst, daß mit einer Hohlkörperwand ein Magnetkompaß mit vertikaler Kompaßachse fest verbunden ist und daß im Umgebungsbereich des Kompasses am Hohlkörper oder einem Ansatz des Hohlkörpers oder am Kompaß selbst mindestens eine Markierung vorgesehen ist, deren die Kompaßachse enthaltende Vertikalebene zu einem Paar Bodenkanten des Hohlkörpers rechtwinklig steht.

Bei vergleichsweise kleinen Hohlkörpern ist der Kompaß vorzugsweise an einer äußeren, sich an eine untere Wandkante anschließende Horizontallasche, bei grösseren begeh baren Hohlkörpern, insbesondere Zelten ist er vorzugsweise an einer von mindestens einer Bodenkante des Hohlkörpers nach innen weisenden Horizontallasche angebracht.

Mit der Erfindung wird erreicht, daß die Position des Kompasses bezüglich des pyramidenförmigen Hohlkörpers ein für allemal festliegt, so daß der Hohlkörper und der Kompaß eine zusammenhängende Einheit bilden, die eine einheitliche Verschwenkung um die Pyramidenachse auch dann erlaubt, wenn der Hohlkörper ein maßgebliches Gewicht hat und des Einsatzes beider Hände bedarf. Es wäre zwar denkbar, in einer Pyramidenfläche eine Ausnehmung vorzusehen und den Kompaß in diese Ausnehmung so einzusetzen, daß die Verlängerung der Pyramidenfläche den Kompaß halbiert, jedoch wäre die Energie-Sammelfunktion des pyramidenförmigen Hohlkörpers mindestens geschwächt.Der Einbau eines Kugelkompasses in eine Hohlkörperwand bringt jedoch die erwünschten Handhabungsvorteile, so daß diese Ausführung zur Erfindung gehört. Vorgezogen wird jedoch die Ausführung, bei der an dem pyramidenförmigen Hohlkörper ein Ansatz oder eine Lasche vorgesehen ist, auf der der Kompaß befestigt ist. Mit dieser Ausführung wird erreicht, daß die Pyramidenflächen unterbrechungsfrei sind.
Dies ist insbesondere wichtig für die Bodenkanten der Pyramidenflächen. Die ganze Pyramide bleibt als solche unverändert erhalten und der vorzugsweise an der Bodenkante
einer Pyramidenfläche anschließende Ansatz oder Lappen
unterbricht die Pyramidenkante nicht.

Die Erfindung ermöglicht es, die genannten Gegenstände
oder Lebewesen auf einer passenden Horizontalfläche zu
positionieren und dann den pyramidenförmigen Hohlkörper
darüber zu stülpen und diesen anschließend mit zwei seiner parallelen Bodenkanten in Nord-Süd-Richtung mit
einer kurzzeitigen Einstellbewegung exakt und fehlerfrei
einzustellen.

Eine Weiterbildung der Erfindung besteht darin, daß die
Markierung oder Markierungen auf der Lasche angebracht,
insbesondere aufgedruckt sind. Erfahrungsgemäß reicht es
nämlich aus, daß der pyramidenförmige Hohlkörper aus Karton besteht. Aus solchem Kartonmaterial kann der Hohlkörperzuschnitt gestanzt und in bekannter Weise zusammengesetzt werden. Der Zuschnitt enthält die Befestigungslasche für den Kompaß, die über eine Bodenkante mit einer
Pyramidenwand verbunden ist, wobei diese Bodenkante eine
Biegelinie bildet. Die Lasche legt sich dann selbsttätig
in die Basisebene der Pyramide. Wesentlich ist, daß bei
der Befestigung des Kompasses darauf geachtet wird, daß die Markierung und die Kompaßachse eine Ebene bilden, die mit einer der benachbarten Hohlkörper-Bodenkanten einen rechten Winkel bildet oder parallel dazu liegt.

Eine weitere Ausgestaltung besteht darin, daß die Lasche dickwandiger ausgebildet ist, beispielsweise aus Holz besteht und daß der Kompaß in einer, eine Tiefe gleich der der Kompaßhöhe aufweisenden Ausnehmung der Lasche eingesetzt ist. Die Kompaßoberfläche liegt damit mit der Oberfläche der Lasche etwa bündig und die Markierung läßt sich noch einfacher z.B. in Form einer Kerbe etwa in gleicher Höhe wie die Kompaßnadel anordnen.

Alternativ besteht eine Ausgestaltung der Erfindung darin, daß der durchsichtige Kompaßdeckel eine zur Kompaßachse radial verlaufende Markierung aufweist. Gemäß dieser Ausgestaltung braucht am Hohlkörper bzw. am Hohlkörperansatz oder -lasche keine Markierung mehr vorgesehen zu werden, und es wird sogar noch an Ablesegenauigkeit gewonnen, weil die Kompaßnadel lediglich mit dem radialen Markierungsstrich zur Deckung gebracht werden muß. Auch hier ist es unabdingbar, daß bei der Montage des Kompasses auf die Positionsgenauigkeit der Markierung bezüglich der Pyramidenbodenkanten geachtet wird. Die Markierung kann aber auch an einer anderen Stelle des Kompasses angebracht sein, und hier besteht

0168513

eine Abwandlung darin, daß die Markierung am Rand des Kompaßgehäuses vorgesehen ist.

Schließlich besteht noch eine Weiterbildung der Erfindung
darin, daß im Hohlkörper ein mit diesem unzusammenhängender
Boden in Form eines Pyramidenstumpfes angeordnet ist,
dessen Basisfläche gleich der geometrischen Basisfläche
des Hohlkörpers ist, dessen äußere Pyramidenflächen parallel
und dicht benachbart zu den Innenflächen der Hohlkörperwände liegen und dessen Höhe im Bereich von 10 % bis 30 %
der Hohlkörperhöhe liegt. Dieser Boden kann aus Vollmaterial oder in Form eines hohlen Topfes ausgebildet sein
und es ist nur wesentlich, daß die obere Deckfläche des
Bodens die lichte Innenhöhe des pyramidenförmigen Hohlkörpers verkleinert, ohne dessen geometrische, regelmäßige Pyramidenform zu verändern. Dieser Boden hat den
Vorteil, daß kleinere Gegenstände, die erfindungsgemäß
mit kosmobiologischer Energie aufgeladen werden sollen,
im optimalen Wirkungsbereich positioniert werden können,
dieser liegt im angegebenen Höhenbereich, der bei besonders kleinen Gegenständen als zwischen 20 % bis 33 %
der gesamten Pyramidenhöhe noch enger eingegrenzt werden kann.

Zur Erzielung optimaler Energie-Sammelergebnisse liegt
das Verhältnis von Höhe des Hohlkörpers zur Länge einer
Bodenkante im Bereich von 0,7 bis 0,9 und beträgt vorzugsweise etwa 0,8.

Der Hohlkörper ist außen vorzugsweise farbig gehalten,
und zwar haben alle vier Pyramidenflächen dieselbe Farbe.
Die Farbe rot hat sich zur Erzielung einer anregenden
Wirkung erwiesen. Die Farbe blau sammelt dagegen Beruhigungsenergie.

Der Hohlkörper besteht erfindungsgemäß aus einem dünnwandigen Material. Außer Karton kann der Hohlkörper gemäß
einer Ausgestaltung der Erfindung aus Holz, Kunststoff
oder auch Nicht-Eisen-Blech, insbesondere Kupfer bestehen.

Anhand der Zeichnung, die ein Ausführungsbeispiel darstellt, sei die Erfindung näher beschrieben.

Es zeigt:

Fig. 1 eine perspektivische Ansicht der neuartigen Einrichtung zum Sammeln kosmobiologischer Energie,

Fig. 2 eine vergrößerte perspektivische Ansicht eines
Details der Figur 1 und

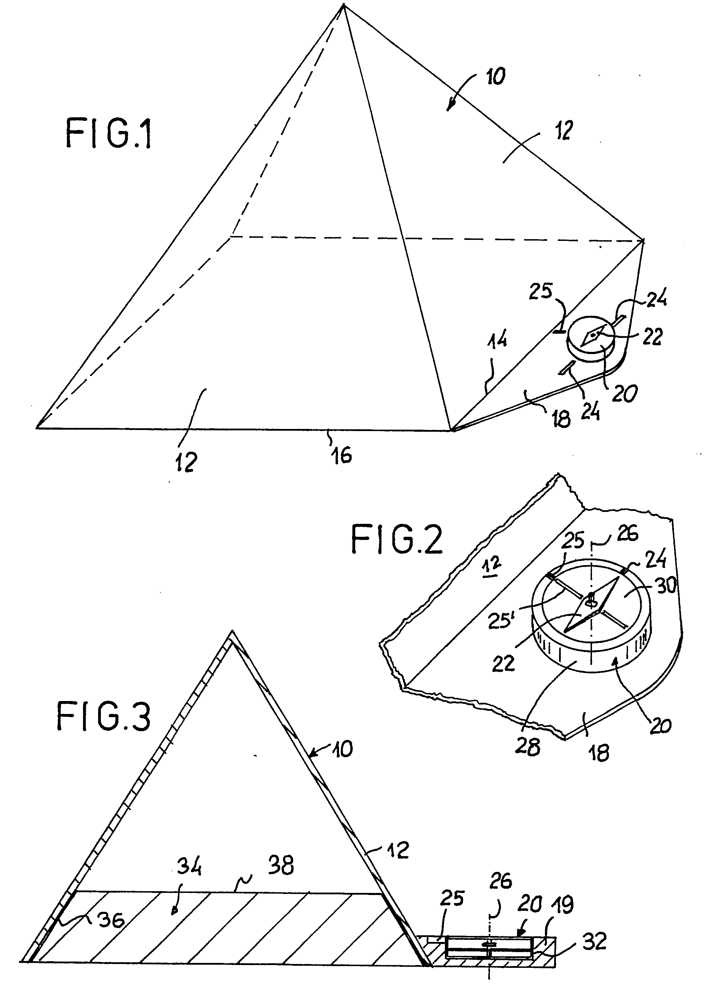

Figu 3 eine mittlere vertikale Schnittansicht durch die
Einrichtung gemäß Figur 1.

Der allgemein mit 10 bezeichnete Hohlkörper ist dünnwandig und besteht aus einem Kartonzuschnitt. Der Hohlkörper weist vier dreieckförmige Seitenflächen auf, die gleichschenklig sind und die im zusammengesetzten Zustand eine unten offene, regelmäßige Pyramide mit quadratischem Grundriß bilden. In einem Ausführungsbeispiel beträgt die Höhe der Pyramide 187 mm, die Bodenkantenlänge 236 mm und die sich daraus ergebende Seitenkantenlänge 250 mm. Für die Funktion kommt es aber nicht auf die absoluten Grundrißmaße an. Wichtig ist vielmehr, daß das Verhältnis von Höhe zu Bodenkantenlänge der Pyramide etwa 0,8 beträgt. Im Ausführungsbeispiel ist dieses Verhältnis 0,79. Die Pyramide 10 ist mit den vier Pyramidenwänden 12 umfangsmäßig vollkommen geschlossen und lediglich auf der Basisseite offen.

An einer Bodenkante 16 der Pyramidenfläche 12 hängt eine Lasche 18. Die Bodenkante 14 bildet eine Faltlinie zwischen der Pyramidenfläche 12 und der Lasche 18. Ist die Pyramide 10 auf eine ebene Unterlage gestellt, so liegt die Lasche 18 horizontal auf dieser Unterlage. Die Lasche 18 ist ebenfalls etwa dreieckförmig. Die Basislänge des Dreiecks ist gleich der Basislänge einer dreiseitigen Pyramidenfläche. Die der Basis der dreieckigen Lasche 18 gegenüberliegende Ecke ist vorzugsweise abgerundet. Die Lasche 18 ist somit in der Längsmitte am breitesten. In diesem Bereich ist auf der Lasche 18 ein einfacher Magnetkompaß 20 befestigt, der z.B. eine Magnetnadel 22 aufweist, aber auch jedes andere magnetische Anzeige-Element haben kann. Der Kompaß 20 hat ein kreiszylindrisches Gehäuse und seine Bodenfläche ist auf der Lasche 18 unlösbar angeklebt. Unmittelbar angrenzend an den Kompaß 20 ist auf der Lasche 18 eine Markierung 24 angebracht, im Ausführungsbeispiel aufgedruckt und nun ist wichtig, daß die relative Lage des Kompasses 20 zur Markierung 24 so gewählt ist, daß die die Kompaßachse 26 (Fig. 2) enthaltende und durch die Markierung 24 laufende Ebene genau parallel zur Bodenkante 14 der benachbarten Pyramidenwand 12 verläuft. Im Ausführungsbeispiel ist eine zweite Markierung 24 in dieser vorher definierten Ebene auf der Lasche 18 angeordnet. Die beiden Markierungen 24, 24 können auch eine durchgehende Linie bilden, die die präzisie Montage des Kompasses 20 erleichtert, weil die Kompaßmitte auf dieser Linie 24 liegen muß.

Die Handhabung der vorstehend beschriebenen Einrichtung 10 ist einfach. Der pyramidenförmige Körper 10 wird über die aufzuladenden Gegenstände gestülpt, die auf einer ebenen Unterlage steht und je nach Größe des Hohlkörpers 10 mit einer oder mit beiden Händen um die geometrische vertikale Pyramidenachse so gedreht, daß die Magnetnadel 22 mit der oder den Markierungen 24 zur Deckung kommt. Die Bodenkante 14 zeigt dann in Nord-Süd-Richtung.

Anstelle der Markierung 24 kann benachbart des Umfanges
des Kompasses 20 auch eine Markierung 25 auf der Lasche 18 angeordnet sein, die ebenfalls in einer die Kompaßachse 26 enthaltenden Vertikalebene liegt, die jedoch nicht wie die Markierungen 24 zur Bodenkante 14
parallel, sondern rechtwinklig dazu liegt. Wird die
Markierung 25 zum Ausrichten des Hohlkörpers 10 benutzt,
so wird dieser so gedreht, daß die Magnetnadel 22 mit
der Markierung 25 fluchtet. In diesem Fall zeigen die
zur Bodenkante 14 rechtwinklig liegenden Bodenkanten 16
in Nord-Süd-Richtung.

In Figur 2 ist eine Abwandlung insofern gezeigt, als
der Kompaß 20 einen Gehäusering 28 aufweist, auf dem eine
der beiden Markierungen 24, 25 angebracht ist. Bei dieser-
Ausbildung braucht die Lasche 18 oder ein entsprechender
Ansatz des Hohlkörpers 10 keine Markierungen aufzuweisen.
Die Handhabung ist dieselbe wie bei der Ausführung gemäß
Figur 1.

Anstelle der Markierung 25 auf dem Rand des Ringes 28
ist in Figur 2 eine Alternative insofern dargestellt,
als der Deckel 30 des Kompasses 20 eine Markierung 25'
aufweist, die als die Kompaßachse 26 kreuzende Radiallinie ausgebildet ist und auf den Deckel 30 aufgedruckt
oder eingraviert sein kann. Es versteht sich, daß jeweils eine der Markierungen für die Handhabung ausreichend ist.

Während bei der Ausführung gemäß Figur 1 der pyramidenförmige Hohlkörper 10 mit Lasche 18 aus Karton hergestellt ist, zeigt Figur 3 einen entsprechenden pyramidenförmigen Hohlkörper 10, der aus Holz hergestellt ist
und der einen vergleichsweise dickwandigen Ansatz 19 im
Bodenbereich einer Hohlkörperwand 12 aufweist. Der Boden des Ansatzes 19 liegt dabei in der geometrischen
Basisfläche des Hohlkörpers 10.In diesem Ansatz 19 befindet sich eine Ausnehmung 32, in die der Kmpaß 20 so
eingepaßt ist, daß er oberseitig mit dem Ansatz 19 bündig liegt. Zur benachbarten Pyramidenwand 12 hin
schließt sich an die Ausnehmung 32 eine in der Oberfläche des Ansatzes 19 eingearbeitete Rille 25 an, die
wiederum in der die Kompaßachse 26 enthaltenden Vertikalebene liegt, welche zur benachbarten Bodenkante rechtwinklig und zu den daran rechtwinklig anschließenden
Bodenkanten parallel liegt.

Im pyramidenförmigen Hohlkörper 10 befindet sich ein Boden 34, der mit dem Hohlkörper 10 nicht zusammenhängt,
der aber eine pyramidenstumpfförmige Gestalt hat, deren
Parameter denjenigen des Hohlkörpers 10 entsprechen, so
daß die Basisfläche des Bodens 34 gleich der inneren Basisfläche des Hohlkörpers 10 ist und die trapezförmigen
Seitenflächen 36 die Innenflächen der Hohlkörperseitenwände 12 mindestens nahezu vollflächig berühren. Der
Boden 34 hat eine zur Basisfläche parallele Deckfläche 38, die von der Basisfläche einen Abstand im Bereich von
10 % bis 30 % der mittleren Hohlkörperhöhe hat. Im Ausführungsbeispiel liegt das Niveau der Deckfläche 38 auf
21 % der Pyramidenhöhe. Der Boden hat den Zweck, kleinere,
mit Energie aufzuladende Gegenstände in den optimalen
Wirkungsbereich des pyramidenförmigen Hohlkörpers zu
bringen. Dieser liegt im Bereich von 10 % bis etwa 30 %
der Pyramidenhöhe. Obwohl der Boden 34 als Vollmaterialkörper dargestellt ist, versteht sich, daß er ebenfalls
aus einem unten offenen Hohlkörper bestehen kann.

Ergänzend zu den eingangs genannten kosmobiologischen
Wirkungen sei erwähnt, daß erwiesener Maßen rohes und
auch gegartes Fleisch über einen längeren Zeitraum unter die Pyramide gelegt wurde. Das Fleisch zeigte keine Verwesungs- oder Fäulnismerkmale sondern lediglich
eine leichtere Schrumpfung. Desgleichen wurden Versuche mit frischer Milch unternommen. Frische Milch,
die längere Zeit unter der Pyramide stand, zeigte keine Spur von Säuerung.

Bei einem großen begehbaren Hohlkörper, insbesondere
in Form eines Zeltes, ist der Kompaß in einer
Bodenlasche im Zeltinneren insbesondere an einem
Zwickel in einer Hohlkörperecke angebracht.

– 1 –

Patentansprüche
_____


1. Einrichtung zum Sammeln von kosmobiologischer Energie, bestehend aus einem Hohlkörper (10) in Form einer unten offenen, regelmäßigen Pyramide mit quadratischem Grundriß, dadurch gekennzeichnet, daß mit einer Hohlkörperwand (12) ein Magnetkompaß (20) mit vertikaler Kompaßachse (26) fest verbunden ist und daß im Umgebungsbereich des Kompasses (20) am Hohlkörper (10) oder einem Ansatz (18; 19) des Hohlkörpers oder am Kompaß (20) selbst mindestens eine Markierung (24; 25; 25') vorgesehen ist, deren die Kompaßachse (26) enthaltende Vertikalebene zu einem Paar Bodenkanten (14; 16) des Hohlkörpers (10) rechtwinklig steht.


2. Einrichtung nach Anspruch 1, dadurch gekennzeichnet, daß sich an der Bodenkante (14) einer Hohlkörperwand (12) eine äußere Lasche (18; 19) anschließt, auf der der Kompaß (20) befestigt ist.

0168513

3. Einrichtung nach Anspruch 2, dadurch gekennzeichnet, daß die Markierung (24; 25) auf der Lasche (18; 19) angebracht ist.

4. Einrichtung nach Anspruch 2 oder 3, dadurch gekennzeichnet, daß der Kompaß (20) in einer, eine Tiefe gleich der Kompaßhöhe aufweisenden Ausnehmung (32) der Lasche (19) eingesetzt ist.

5. Einrichtung nach Anspruch 1 oder 2, dadurch gekennzeichnet, daß der durchsichtige Kompaßdeckel (30) eine zur Kompaßachse (26) radial verlaufende Markierung (25') aufweist.

6. Einrichtung nach Anspruch 1 oder 2, dadurch gekennzeichnet, daß die Markierung (24; 25) am Rand des Kompaßgehäuses (28) vorgesehen ist.

7. Einrichtung nach einem der Ansprüche 1 - 6, dadurch gekennzeichnet, daß im Hohlkörper (10) ein mit diesem unzusammenhängender Boden (34) in Form eines Pyramidenstumpfes angeordnet ist, dessen Basisfläche gleich der geometrischen Basisfläche des Hohlkörpers (10) ist, dessen äußere Pyramidenflächen parallel und dicht benachbart zu den Innenflächen der Hohlkörperwände (12) liegen und dessen Höhe im Bereich von 10 bis 30 % der Hohlkörperhöhe liegt.

8. Einrichtung nach einem der Ansprüche 1 - 7, dadurch gekennzeichnet, daß das Verhältnis von Höhe des Hohlkörpers (10) zur Länge einer Bodenkante (14; 16) im Bereich von 0,7 bis 0,9 liegt und vorzugsweise etwa 0,8 beträgt.

9. Einrichtung nach einem der Ansprüche 1 - 8, dadurch gekennzeichnet, daß der Hohlkörper (10) dünnwandig ausgebildet ist.

10. Einrichtung nach Anspruch 9, dadurch gekennzeichnet, daß der Hohlkörper (10) aus Karton, Holz, Kunststoff oder Nicht-Eisen-Blech wie Kupfer besteht.

FIG.1

FIG.2

FIG.3

0168513

1/1

Europäisches
Patentamt

**EUROPÄISCHER RECHERCHENBERICHT**

0168513
Nummer der Anmeldung

EP 84 10 8449

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.4) |
|---|---|---|---|
| X | FR-A- 718 425 (E. STETTNER) * Seite 2, Zeilen 56-76; Abbildungen 1,2 * | 1-4 | A 61 N 1/16 |
| | --- | | |
| A | FR-A-2 264 406 (G. FANTUZZI) * Seite 1, Zeilen 1-16; Seite 2, Zeilen 8-24; Abbildung 4 * | 1,9,10 | |
| | --- | | |
| A | FR-A-1 584 210 (R. JACQUET) | | |
| | ----- | | |

| RECHERCHIERTE SACHGEBIETE (Int. Cl.4) |
|---|
| A 61 N 1/00 |

Der vorliegende Recherchenbericht wurde für alle Patentansprüche erstellt.

| Recherchenort DEN HAAG | Abschlußdatum der Recherche 15-03-1985 | Prüfer ERRANI C. |
|---|---|---|